# FASCICULE DE BREVET EUROPEEN

(11) **EP 3 183 552 B1**
(45) Date de publication et mention de la délivrance du brevet: **26.09.2018**
(21) Numéro de dépôt: 15756130.9
(22) Date de dépôt: 18.08.2015
(51) Int. Cl.: G01N 33/18, E03B 3/03, G01N 1/18, G01W 1/14

(54) **DISPOSITIF DE COLLECTE SÉQUENTIELLE D'EAU DE PLUIE, NOTAMMENT EN VUE DE L'ÉTUDE DE L'ÉVOLUTION DE LA RADIOACTIVITÉ DES EAUX DE PLUIE**
VORRICHTUNG ZUR SEQUENZIELLEN SAMMLUNG VON REGENWASSER, INSBESONDERE IM HINBLICK AUF DIE UNTERSUCHUNG DER VERÄNDERUNG DER RADIOAKTIVITÄT VON REGENWASSER
DEVICE FOR SEQUENTIALLY COLLECTING RAINWATER, IN PARTICULAR WITH A VIEW TO STUDYING THE VARIATION IN THE RADIOACTIVITY OF RAINWATER

(30) Priorité: 22.08.2014 FR 1457941
(43) Date de publication de la demande: 28.06.2017
(73) Titulaire: Institut de Radioprotection et de Sûreté Nucléaire, 92260 Fontenay aux Roses (FR)
(72) Inventeur: MARO, Denis, 50100 Cherbourg-Octeville (FR); LAGUIONIE, Philippe, 50700 Valognes (FR)
(74) Mandataire: Lavoix
(86) Numéro de dépôt international: PCT/EP2015/068908
(87) Numéro de publication internationale: WO 2016/026836

(56) Documents cités:
- EP-A1- 0 656 448
- WO-A1-99/09263
- WO-A1-2009/044927
- FR-A1- 2 912 162

## Description

La présente invention concerne un dispositif de collecte séquentielle d'eau de pluie, notamment en vue de l'étude de l'évolution de la radioactivité des eaux de pluie.

On connait déjà, dans l'état de la technique, un dispositif de collecte comportant un réceptacle de grande dimension, qui se remplit progressivement au gré des pluies. Puisque toutes les pluies sont collectées dans un même réceptacle, un tel dispositif de collecte ne permet pas d'étudier une évolution de l'activité des eaux de pluie au cours du temps.

Afin d'étudier cette évolution, on connaît également, dans l'état de la technique, un dispositif de collecte, dit séquentiel, comportant une pluralité de réceptacles de collectes destinés à recevoir de l'eau de pluie, ces réceptacles pouvant être ouverts ou fermés indépendamment. Toutefois, un tel dispositif de collecte est particulièrement onéreux, et il nécessite une source d'énergie pour alimenter des moyens d'ouverture et de fermeture des réceptacles. Cette énergie est généralement de l'électricité délivrée sur par un réseau électrique, si bien qu'un tel dispositif de collecte présente des contraintes liées à sa connexion avec ce réseau électrique, qui limitent ses possibilités d'installation dans l'environnement.

On connait également, notamment d'après WO 2009/044927, un dispositif de collecte d'eau de pluie similaire à celui du préambule de la revendication 1.

Le document FR 2 912 162 décrit par ailleurs un dispositif de collecte d'eau de pluie comportant plusieurs réceptacles empilés verticalement.

L'invention a notamment pour but de remédier aux inconvénients précités, en fournissant un dispositif de collecte d'eau de pluie de type séquentiel ne nécessitant pas de source d'énergie, tout en restant économique.

A cet effet, l'invention a notamment pour objet un dispositif de collecte d'eau de pluie, comportant une pluralité de réceptacles de collecte destinés à recevoir de l'eau de pluie, chaque réceptacle de collecte comportant une paroi circonférentielle et une paroi inférieure, dans lequel :
- les réceptacles de collecte sont empilés, formant un empilement le long d'un axe vertical,
- chaque réceptacle agencé au-dessus d'un autre réceptacle adjacent comporte une ouverture d'écoulement d'eau ménagée dans sa paroi inférieure, cette ouverture d'écoulement débouchant dans cet autre réceptacle adjacent,
- chaque réceptacle agencé en-dessous d'un autre réceptacle adjacent loge un élément d'obturation flottant, mobile le long de l'axe vertical entre une position basse et une position haute en fonction de la quantité d'eau dans ce réceptacle, l'élément d'obturation présentant une forme complémentaire de celle de l'ouverture d'écoulement d'eau du réceptacle adjacent agencé au-dessus, obturant cette ouverture d'écoulement en position haute, et
- chaque réceptacle logeant un élément d'obturation comporte des moyens magnétiques de maintien de l'élément d'obturation en position haute.

Les réceptacles de collecte étant empilés, l'eau de pluie s'écoule à travers ces réceptacles, notamment à travers les ouvertures d'écoulement, jusqu'à un réceptacle inférieur agencé le plus bas. Au fur et à mesure que ce réceptacle inférieur se remplit, l'élément d'obturation flottant logé dans ce réceptacle se déplace depuis sa position basse jusqu'à sa position haute. En position haute, l'ouverture d'écoulement débouchant dans ce réceptacle inférieur est obturée, et l'eau de pluie remplit maintenant un nouveau réceptacle. Ainsi, à terme, les réceptacles comportent des eaux de pluie correspondant à des précipitations différentes, qui ne se mélangent pas.

Il apparaît donc clairement que le dispositif de collecte selon l'invention permet d'étudier l'évolution de l'état des eaux de pluie.

La fermeture des réceptacles étant réalisée de manière mécanique, par les éléments d'obturation flottants, le dispositif de collecte selon l'invention ne nécessite aucune source d'alimentation.

Enfin, le dispositif de collecte selon l'invention présente une structure simple, et donc particulièrement économique.

Un dispositif de collecte selon l'invention peut comporter en outre l'une ou plusieurs des caractéristiques suivantes, prises seules ou selon toutes combinaisons techniquement envisageables.
- La pluralité de réceptacles comporte : un réceptacle supérieur, agencé en haut de l'empilement, dont la paroi inférieure comporte une ouverture d'écoulement, un réceptacle inférieur, agencé en bas de l'empilement, logeant un élément d'obturation, et au moins un réceptacle intermédiaire, agencé entre le réceptacle supérieur et le réceptacle inférieur dans la direction de l'axe vertical, présentant d'une part une paroi inférieure comportant une ouverture d'écoulement et logeant d'autre part un élément d'obturation flottant.
- Les réceptacles sont assemblés de manière détachable les uns aux autres, par exemple par vissage ou encliquetage.
- Chaque réceptacle comporte un canal de vidage traversant sa paroi circonférentielle à proximité de sa paroi inférieure, chaque canal de vidage étant muni d'une vanne.
- La paroi inférieure de chaque réceptacle présente une concavité, dans laquelle débouche le canal de vidage de ce réceptacle.
- Un joint d'étanchéité est agencé entre chaque élément d'obturation et chaque ouverture d'écoulement complémentaire, le joint d'étanchéité étant porté par l'élément d'obturation ou par un pourtour de l'ouverture d'écoulement.
- Les moyens magnétiques comportent un élément annulaire, logé dans le réceptacle à proximité de la paroi inférieure du réceptacle adjacent situé immédiatement au-dessus, l'un parmi l'élément annulaire et l'élément d'obturation étant en matériau magnétique et l'autre en matériau ferromagnétique.
- Chaque élément d'obturation présente une forme de cône, et chaque réceptacle logeant un élément d'obturation présente un élément présentant une ouverture tronconique de réception de l'élément d'obturation en position haute.
- La pluralité de réceptacles comporte un réceptacle supérieur, agencé en haut de l'empilement, comprenant un canal d'évacuation de trop plein traversant sa paroi circonférentielle à une extrémité supérieure de ce réceptacle supérieur.

L'invention sera mieux comprise à la lecture de la description qui va suivre, donnée uniquement à titre d'exemple et faite en se référant aux figures annexées parmi lesquelles :
- la figure 1 est une vue schématique en coupe d'un dispositif de collecte d'eau de pluie selon un exemple de mode de réalisation de l'invention, dans lequel les réceptacles de collecte sont ouverts ;
- la figure 2 est une vue schématique en coupe d'un détail du dispositif de collecte de la figure 1, représentant un réceptacle de collecte refermé.

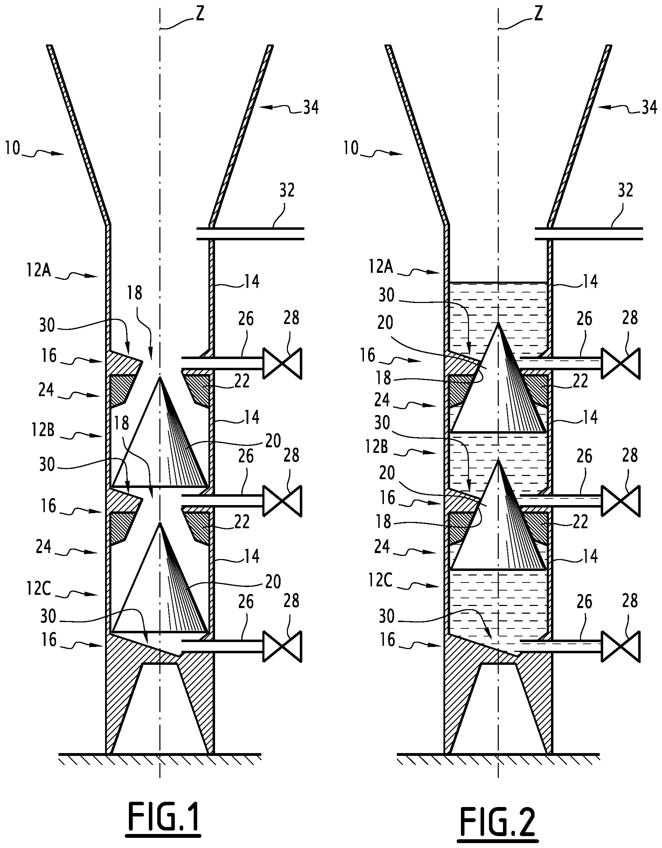

On a représenté, sur la figure 1, un dispositif 10 de collecte d'eau de pluie selon un exemple de mode de réalisation de l'invention.

Le dispositif de collecte 10 comporte une pluralité de réceptacles 12A, 12B, 12C, destinés à recevoir de l'eau de pluie. Chaque réceptacle de collecte 12A, 12B, 12C comporte une paroi circonférentielle 14, et une paroi inférieure 16. Chaque réceptacle de collecte 12A, 12B, 12C présente par exemple une forme général cylindrique à section circulaire, s'étendant le long d'un axe Z.

Les réceptacles de collecte 12A, 12B, 12C sont empilés de manière à former un empilement le long de l'axe Z. L'axe Z sera, dans la présente description, appelé « axe vertical » car, pour une utilisation normale du dispositif de collecte 10, les réceptacles de collecte 12A, 12B, 12C doivent être empilés verticalement.

Dans la présente description, les termes « supérieur », « inférieur », « dessus », « dessous », « haut » et « bas » sont définis le long de cet axe vertical Z, avec leur sens classique.

Dans l'exemple représenté, la pluralité de réceptacles 12A, 12B, 12C comporte un réceptacle supérieur 12A, agencé en haut de l'empilement, un réceptacle intermédiaire 12B, et un réceptacle inférieur 12C, agencé en bas de l'empilement. Le réceptacle intermédiaire 12B est donc agencé entre le réceptacle supérieur 12A et le réceptacle inférieur 12C dans la direction de l'axe vertical Z.

Conformément à une variante non représentée, l'empilement pourrait comporter au moins deux réceptacles intermédiaires 12B, identiques, empilés entre le réceptacle supérieur 12A et le réceptacle inférieur 12C.

Conformément à une autre variante non représentée, l'empilement pourrait ne pas comporter de réceptacle intermédiaire 12B, mais uniquement un réceptacle supérieur 12A et un réceptacle inférieur 12C.

Plus particulièrement, le nombre de réceptacles est choisi en fonction du nombre de prélèvements d'eau de pluie que l'on souhaite effectuer.

Avantageusement, les réceptacles 12A, 12B, 12C sont assemblés de manière détachable les uns aux autres, par exemple par vissage ou encliquetage. Chaque réceptacle 12A, 12B, 12C forme donc un module, si bien que le dispositif de collecte 10 est modulaire et peut présenter de manière simple un nombre souhaité de réceptacles.

Afin de permettre un écoulement des eaux de pluie jusqu'au réceptacle inférieur 12C, chaque réceptacle 12A, 12B agencé au-dessus d'un autre réceptacle adjacent 12B, 12C comporte une ouverture 18 d'écoulement d'eau ménagée dans sa paroi inférieure 16, cette ouverture d'écoulement 18 débouchant dans ledit autre réceptacle adjacent 12B, 12C. Plus particulièrement, le réceptacle supérieur 12A, ainsi que chaque réceptacle intermédiaire 12B, comporte une telle ouverture d'écoulement 18 dans sa paroi inférieure 16.

En revanche, le réceptacle inférieur 12C ne comporte pas d'ouverture d'écoulement dans sa paroi inférieure 16. En variante, le réceptacle inférieur 12C présente une structure identique à celle du réceptacle intermédiaire 12B, auquel cas son ouverture d'écoulement 18 est obturée, par exemple par un socle sur lequel est disposé ce réceptacle inférieur 12C.

Par ailleurs, chaque réceptacle 12B, 12C agencé en dessous d'un autre réceptacle adjacent 12A, 12B loge un élément d'obturation 20 flottant. Cet élément d'obturation flottant 20 est mobile le long de l'axe vertical Z entre une position basse et une position haute, en fonction de la quantité d'eau dans ce réceptacle 12B, 12C. L'élément d'obturation 20 présente une forme complémentaire de celle de l'ouverture d'écoulement d'eau 18 du réceptacle adjacent 12A, 12B agencé directement au-dessus, de sorte qu'il est susceptible d'obturer cette ouverture d'écoulement 18 lorsqu'il se trouve en position haute, comme cela est représenté sur la figure 2.

En revanche, l'élément d'obturation 20 présente une forme autorisant l'écoulement de l'eau vers le réceptacle adjacent 12B, 12C agencé au-dessous lorsqu'il se trouve dans sa position basse. A cet effet, l'élément d'obturation 20 peut présenter des rainures de passage d'eau dans sa partie basse, présenter des pieds surélévant l'élément d'obturation 20 en position basse, ou présenter tout autre moyen envisageable pour autoriser l'écoulement de l'eau.

Avantageusement, le dispositif de collecte 10 comporte des moyens de guidage en translation de chaque élément d'obturation 20 le long de l'axe vertical Z. Ces moyens de guidage peuvent prendre toute forme envisageable.

Par exemple, dans l'exemple représenté chaque élément d'obturation 20 présente une forme de cône, et chaque réceptacle 12B, 12C logeant un tel élément d'obturation 20 présente un élément annulaire 22, présentant une ouverture tronconique de guidage de l'élément d'obturation 20 et de réception de cet élément d'obturation 20 en position haute. Cet élément annulaire 22 permet à la fois de guider l'élément d'obturation 20 lorsqu'il se déplace vers sa position haute et d'optimiser l'étanchéité entre les deux réceptacles adjacents, en augmentant la surface de contact entre l'élément d'obturation 20 en cône et l'élément annulaire 22.

Avantageusement, un joint d'étanchéité (non représenté) est agencé entre chaque élément d'obturation 20 et chaque ouverture d'écoulement complémentaire 18. Le joint d'étanchéité est par exemple porté par l'élément d'obturation 20, ou en variante par un pourtour de l'ouverture d'écoulement 18, par exemple par l'élément annulaire 22.

Chaque réceptacle 12B, 12C logeant un élément d'obturation 20 comporte des moyens magnétiques de maintien de l'élément d'obturation 20 en position haute, assurant le maintien de la fermeture de ce réceptacle 12B, 12C.

Les moyens magnétiques 24 comportent par exemple un élément de maintien, logé dans le réceptacle 12B, 12C à proximité de la paroi inférieure du réceptacle adjacent situé immédiatement au-dessus, l'un parmi l'élément de maintien et l'élément d'obturation 20 étant en matériau magnétique, et l'autre en matériau ferromagnétique.

Dans l'exemple représenté, l'élément de maintien est formé par l'élément annulaire 22.

Par exemple, l'élément d'obturation 20 est en acier non-inoxydable, recouvert d'un polymère protégeant l'acier de la rouille.

Il apparaît clairement que le réceptacle le plus bas qui n'est pas encore rempli se remplit au fur et à mesure des pluies s'écoulant dans les réceptacles, entraînant la montée de son élément d'obturation 20 jusqu'à sa position haute. Une fois cette position haute atteinte, cet élément d'obturation 20 obture l'ouverture d'écoulement débouchant dans ce réceptacle, si bien que ce réceptacle est ainsi refermé et isolé des autres réceptacles. C'est ensuite le réceptacle immédiatement supérieur qui sera rempli de la même manière.

Les réceptacles sont donc remplis au fur et à mesure, les uns après les autres, jusqu'à ce que le réceptacle supérieur 12A soit également rempli.

Puisque les réceptacles sont remplis les uns après les autres, ils comportent des eaux de pluie correspondant à différentes précipitations, ce qui permet d'observer une évolution de l'état des eaux de pluie.

De manière optionnelle, le dispositif de collecte d'eau 10 est associé à une station météorologique (non représentée), qui relève les précipitations et permet donc de déduire les dates des précipitations correspondant aux différents prélèvements.

Afin de prélever de l'eau de chaque réceptacle 12A, 12B, 12C, chaque réceptacle comporte un canal de vidage 26 traversant sa paroi circonférentielle14 à proximité de sa paroi inférieure 16. Chaque canal de vidage 26 est muni d'une vanne 28, qui est fermée lors de la collecte d'eau de pluie, et ouverte lors d'un prélèvement de l'eau du réceptacle correspondant.

Avantageusement, chaque paroi inférieure 16 présente une concavité 30, dans laquelle débouche le canal de vidage 26. Cette concavité 30 permet d'entraîner l'eau vers le canal de vidage 26, afin d'assurer que toute l'eau du réceptacle est bien évacuée par ce canal de vidage 26.

Une fois l'eau de pluie prélevée, celle-ci est analysée de manière connue en soi.

De manière optionnelle, le réceptacle supérieur 12A comprend un canal d'évacuation de trop-plein 32, traversant sa paroi circonférentielle 14 à une extrémité supérieure de ce réceptacle supérieur 12A. Ainsi, le réceptacle supérieur 12A ne peut pas être rempli au-delà de ce canal d'évacuation 32

Enfin, le dispositif de collecte 10 comporte avantageusement un cône d'entrée 34, agencé au-dessus du réceptacle supérieur 12A, facilitant l'entrée des eaux de pluie dans le dispositif de collecte 10.

On notera que l'invention n'est pas limitée au mode de réalisation précédemment décrit, mais pourrait présenter diverses variantes.

En particulier, chaque élément d'obturation pourrait présenter une forme différente de celle décrite précédemment.

## Revendications

1. Dispositif (10) de collecte d'eau de pluie, comportant une pluralité de réceptacles de collecte (12A, 12B, 12C) destinés à recevoir de l'eau de pluie, chaque réceptacle de collecte (12A, 12B, 12C) comportant une paroi circonférentielle (14) et une paroi inférieure (16), dans lequel :
- les réceptacles de collecte (12A, 12B, 12C) sont empilés, formant un empilement le long d'un axe vertical (Z),
- chaque réceptacle (12A, 12B) agencé au-dessus d'un autre réceptacle adjacent (12B, 12C) comporte une ouverture d'écoulement d'eau (18) ménagée dans sa paroi inférieure (16), cette ouverture d'écoulement (18) débouchant dans cet autre réceptacle adjacent (12B, 12C),
- chaque réceptacle (12B, 12C) agencé en-dessous d'un autre réceptacle adjacent (12A, 12B) loge un élément d'obturation flottant (20), mobile le long de l'axe vertical (Z) entre une position basse et une position haute en fonction de la quantité d'eau dans ce réceptacle (12B, 12C), l'élément d'obturation (20) présentant une forme complémentaire de celle de l'ouverture d'écoulement d'eau (18) du réceptacle adjacent (12A, 12B) agencé au-dessus, obturant cette ouverture d'écoulement (18) en position haute, et
**caractérisé en ce que** chaque réceptacle (12B, 12C) logeant un élément d'obturation (20) comporte des moyens magnétiques de maintien de l'élément d'obturation (20) en position haute.

2. Dispositif de collecte (10) selon la revendication 1, dans lequel la pluralité de réceptacles (12A, 12B, 12C) comporte :
- un réceptacle supérieur (12A), agencé en haut de l'empilement, dont la paroi inférieure (16) comporte une ouverture d'écoulement (18),
- un réceptacle inférieur (12C), agencé en bas de l'empilement, logeant un élément d'obturation (20), et
- au moins un réceptacle intermédiaire (12B), agencé entre le réceptacle supérieur (12A) et le réceptacle inférieur (12C) dans la direction de l'axe vertical (Z), présentant d'une part une paroi inférieure (16) comportant une ouverture d'écoulement (18) et logeant d'autre part un élément d'obturation flottant (20).

3. Dispositif de collecte (10) selon la revendication 1 ou 2, dans lequel les réceptacles (12A, 12B, 12C) sont assemblés de manière détachable les uns aux autres, par exemple par vissage ou encliquetage.

4. Dispositif de collecte (10) selon l'une quelconque des revendications précédentes, dans lequel chaque réceptacle (12A, 12B, 12C) comporte un canal de vidage (26) traversant sa paroi circonférentielle (14) à proximité de sa paroi inférieure (16), chaque canal de vidage (26) étant muni d'une vanne (28).

5. Dispositif de collecte (10) selon la revendication 4, dans lequel la paroi inférieure (16) de chaque réceptacle (12A, 12B, 12C) présente une concavité (30), dans laquelle débouche le canal de vidage (26) de ce réceptacle (12A, 12B, 12C).

6. Dispositif de collecte (10) selon l'une quelconque des revendications précédentes, dans lequel un joint d'étanchéité est agencé entre chaque élément d'obturation (20) et chaque ouverture d'écoulement (18) complémentaire, le joint d'étanchéité étant porté par l'élément d'obturation (20) ou par un pourtour de l'ouverture d'écoulement (18).

7. Dispositif de collecte (10) selon l'une quelconque des revendications précédentes, dans lequel les moyens magnétiques comportent un élément annulaire (22), logé dans le réceptacle (12B, 12C) à proximité de la paroi inférieure (16) du réceptacle adjacent (12A, 12B) situé immédiatement au-dessus, l'un parmi l'élément annulaire (22) et l'élément d'obturation (20) étant en matériau magnétique et l'autre en matériau ferromagnétique.

8. Dispositif de collecte (10) selon l'une quelconque des revendications précédentes, dans lequel chaque élément d'obturation (20) présente une forme de cône, et chaque réceptacle (12B, 12C) logeant un élément d'obturation (20) présente un élément (22) présentant une ouverture tronconique de réception de l'élément d'obturation (20) en position haute.

9. Dispositif de collecte (10) selon l'une quelconque des revendications précédentes, dans lequel la pluralité de réceptacles (12A, 12B, 12C) comporte un réceptacle supérieur (12A), agencé en haut de l'empilement, comprenant un canal (32) d'évacuation de trop plein traversant sa paroi circonférentielle (14) à une extrémité supérieure de ce réceptacle supérieur (12A).

## Patentansprüche

1. Vorrichtung (10) zum Sammeln von Regenwasser, die eine Mehrzahl von zur Aufnahme von Regenwasser geeigneten Sammelbehältern (12A, 12B, 12C) aufweist, wobei jeder Sammelbehälter (12A, 12B, 12C) eine Umfangswand (14) und eine untere Wand (16) aufweist, wobei:
- die Sammelbehälter (12A, 12B, 12C) unter Bildung eines Stapels entlang einer vertikalen Achse (Z) aufeinandergestapelt sind,
- jeder Behälter (12A, 12B), der über einem anderen benachbarten Behälter (12B, 12C) angeordnet ist, eine Strömungsöffnung (18) für Wasser aufweist, die in seiner unteren Wand (16) eingearbeitet ist, wobei diese Strömungsöffnung (18) in diesen anderen benachbarten Behälter (12B, 12C) mündet,
- jeder Behälter (12B, 12C), der unter einem anderen benachbarten Behälter (12A, 12B) angeordnet ist, ein schwimmendes Verschlusselement (20) aufnimmt, das entlang der vertikalen Achse (Z) zwischen einer tiefen Stellung und einer hohen Stellung abhängig von der Wassermenge in diesem Behälter (12B, 12C) beweglich ist, wobei das Verschlusselement (20) eine komplementäre Form zu derjenigen der Strömungsöffnung (18) für Wasser des benachbarten Behälters (12A, 12B), der darüber liegt, aufweist, wodurch diese Strömungsöffnung (18) in der hohen Stellung verschlossen wird, und
**dadurch gekennzeichnet, dass** jeder Behälter (12B, 12C), in dem ein Verschlusselement (20) lagert, magnetische Mittel zum Halten des Verschlusselements (20) in der hohen Stellung aufweist.

2. Sammelvorrichtung (10) nach Anspruch 1, bei der die Mehrzahl von Behältern (12A, 12B, 12C) aufweist:
- einen oberen Behälter (12A), der an der oberen Stelle des Stapels angeordnet ist und dessen untere Wand (16) eine Strömungsöffnung (18) aufweist,
- einen unteren Behälter (12C), der an der unteren Stelle des Stapels liegt und ein Verschlusselement (20) aufnimmt, und
- mindestens einen Zwischenbehälter (12B), der zwischen dem oberen Behälter (12A) und dem unteren Behälter (12C) in Richtung der vertikalen Achse (Z) liegt und einerseits eine unteren Wand (16) aufweist, die eine Strömungsöffnung (18) umfasst, und andererseits ein schwimmendes Verschlusselement (20) aufnimmt.

3. Sammelvorrichtung (10) nach Anspruch 1 oder 2, bei der die Behälter (12A, 12B, 12C) in einer zueinander lösbaren Weise zusammengesetzt sind, beispielsweise durch Verschrauben oder Verrasten.

4. Sammelvorrichtung (10) nach einem beliebigen der vorhergehenden Ansprüche, bei der jeder Behälter (12A, 12B, 12C) einen Entleerungskanal (26) aufweist, der seine Umfangswand (14) in der Nähe seiner unteren Wand (16) durchgreift, wobei jeder Entleerungskanal (26) mit einem Ventil (28) versehen ist.

5. Sammelvorrichtung (10) nach Anspruch 4, bei der die untere Wand (16) jedes Behälters (12A, 12B, 12C) eine Konkavität (30) aufweist, in die der Entleerungskanal (26) dieses Behälters (12A, 12B, 12C) mündet.

6. Sammelvorrichtung (10) nach einem beliebigen der vorhergehenden Ansprüche, bei der eine Dichtung zwischen jedem Verschlusselement (20) und jeder komplementären Strömungsöffnung (18) angeordnet ist, wobei das Dichtungselement von dem Verschlusselement (20) oder von einem Umfang der Strömungsöffnung (18) getragen wird.

7. Sammelvorrichtung (10) nach einem beliebigen der vorhergehenden Ansprüche, bei der die magnetischen Mittel ein Ringelement (22) aufweisen, das in dem Behälter (12B, 12C) in der Nähe der unteren Wand (16) des benachbarten (12A, 12B), unmittelbar darüberliegenden Behälter gelagert ist, wobei das eine der Elemente Ringelement (22) und Verschlusselement (20) aus einem magnetischen Material besteht und das andere ein ferromagnetisches Material ist.

8. Sammelvorrichtung (10) nach einem beliebigen der vorhergehenden Ansprüche, bei der jedes Verschlusselement (20) eine Kegelform aufweist und jeder Behälter (12B, 12C), in dem ein Verschlusselement (20) gelagert ist, ein Element (22) aufweist, das eine kegelstumpfförmige Öffnung zur Aufnahme des Verschlusselements (20) in der hohen Stellung aufweist.

9. Sammelvorrichtung (10) nach einem beliebigen der vorhergehenden Ansprüche, bei der die Mehrzahl von Behältern (12A, 12B, 12C) einen oberen Behälter (12A), der an der oberen Stelle des Stapels angeordnet ist, und einen Überlaufkanal (32) umfasst, der seine Umfangswand (14) an einem oberen Ende dieses oberen Behälters (12A) durchgreift.

## Claims

1. A device (10) for collecting rainwater, including a plurality of collecting receptacles (12A, 12B, 12C) intended to receive rainwater, each collecting receptacle (12A, 12B, 12C) including a circumferential wall (14) and a lower wall (16), wherein:
- the collecting receptacles (12A, 12B, 12C) are stacked, forming a stack along a vertical axis (Z),
- each receptacle (12A, 12B) arranged above another adjacent receptacle (12B, 12C) includes an aperture for water flow (18) made in its lower wall (16), this flow aperture (18) opening into this other adjacent receptacle (12B, 12C),
- each receptacle (12B, 12C) arranged below another adjacent receptacle (12A, 12B) houses a floating obturation element (20), movable along the vertical axis (Z) between a low position and a high position depending on the amount of water in this receptacle (12B, 12C), the obturation element (20) having a shape mating that of the water flow aperture (18) of the adjacent receptacle (12A, 12B) arranged above, obturating this flow aperture (18) in the high position, and
**characterized in that** each receptacle (12B, 12C) housing an obturation element (20) includes magnetic means for maintaining the obturation element (20) in a high position.

2. The collecting device (10) according to claim 1, wherein the plurality of receptacles (12A, 12B, 12C) includes:
- an upper receptacle (12A), arranged at the top of the stack, for the lower wall (16) of which includes a flow aperture (18),
- a lower receptacle (12C), arranged at the bottom of the stack, housing an obturation element (20), and
- at least one intermediate receptacle (12B), arranged between the upper receptacle (12A) and the lower receptacle (12C) in the direction of the vertical axis (Z), having on the one hand a lower wall (16) including a flow aperture (18) and housing on the other hand a floating obturation element (20).

3. The collecting device (10) according to claim 1 or 2, wherein the receptacles (12A, 12B, 12C) are assembled in a detachable way to each other for example by screwing or snap-fastening.

4. The collecting device (10) according to any of the preceding claims, wherein each receptacle (12A, 12B, 12C) includes an emptying channel (26) crossing its circumferential wall (14) in proximity to its lower wall (16), each emptying channel (26) being provided with a valve (28).

5. The collecting device (10) according to claim 4, wherein the lower wall (16) of each receptacle (12A, 12B, 12C) has a concavity (30), into which opens the emptying channel (26) of this receptacle (12A, 12B, 12C).

6. The collecting device (10) according to any of the preceding claims, wherein a seal gasket is arranged between each obturation element (20) and each complementary flow aperture (18), the seal gasket being borne by the obturation element (20) or by a perimeter of the flow aperture (18).

7. The collecting device (10) according to any of the preceding claims, wherein the magnetic means include an annular element (22), housed in the receptacle (12B, 12C) in proximity to the lower wall (16) of the adjacent receptacle (12A, 12B) located immediately above, one from among the annular element (22) and the obturation element (20) being in a magnetic material and the other one in a ferromagnetic material.

8. The collecting device (10) according to any of the preceding claims, wherein each obturation element (20) has a cone shape, and each receptacle (12B, 12C) housing an obturation element (20) has an element (22) having a frusto-conical aperture for receiving the obturation element (20) in a high position.

9. The collecting device (10) according to any of the preceding claims, wherein the plurality of receptacles (12A, 12B, 12C) includes an upper receptacle (12A), arranged at the top of the stack, comprising a channel (32) for discharging an overflow crossing its circumferential wall (14) at an upper end of this upper receptacle (12A).
